(19) Europäisches Patentamt
European Patent Office
Office européen des brevets

(11) **EP 1 598 780 A1**

## (12) EUROPEAN PATENT APPLICATION

(43) Date of publication:
**23.11.2005 Bulletin 2005/47**

(51) Int Cl.7: **G06T 5/40**

(21) Application number: **05252905.4**

(22) Date of filing: **11.05.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **17.05.2004 JP 2004146705**

(71) Applicant: **GE Medical Systems Global Technology Company, LLC
Waukesha, Wisconsin 53188-1696 (US)**

(72) Inventor: **Hagiwara, Akira
Hino-shi Tokyo 191-8503 (JP)**

(74) Representative: **Goode, Ian Roy
London Patent Operation,
General Electric International Inc.,
15 John Adam Street
London WC2N 6LU (GB)**

(54) **Image processing method, image processing system, and x-ray CT system**

(57)     An object of the present invention is to provide an image processing method for handling image information, which contains a plurality of structural image data items, without degradation in image quality even partly. A producing means (210) produces a graph of a cumulative distribution function, which provides for each pixel value a cumulative number of pixels assuming a certain value, using image data contained in a region of processing delineated in tomographic image information. A working-out means (220) works out local standard deviations of pixel values using the graph of the cumulative distribution function. A calculating means (230) calculates a plurality of region identification thresholds on the basis of the smallest standard deviation that is the smallest value among the local standard deviations. A classifying means (240) classifies the local standard deviations, or eventually, the pixels exhibiting the local standard deviations on the basis of the region identification thresholds. Moreover, a selecting means (250) selects weight coefficients, which define a smoothing filter, for each of the categories in to which the pixels are classified based on the region identification thresholds. A processing means (260) applies the weight coefficients to pixels contained in the tomographic image information so as to achieve smoothing.

Fig. 2

**Description**

The present invention relates to an image processing method, an image processing system, and an X-ray CT system that perform different kinds of image processing on respective structural image data items that are contained in image information.

In recent years, digital image information has come to be frequently dealt with even in the field of medicine along with prevalence of X-ray CT systems or the like. In the field of medicine, tomographic image information on a subject is mainly treated as digital image information and proves helpful in examination of the subject. Moreover, the digital image information is subjected to image processing including removal of a noise component because a clear image is required for interpretation.

In the image processing, a spatial filter for smoothing or sharpening is used to remove a noise component or sharpen a boundary on the basis of a difference between spatial-frequency components (refer to, for example, Non-patent Document "Image Data Processing for Scientific Measurement" (Satoshi Kawada, CQ Publishing, April 30, 1994, pp.143-180)).

However, according to the foregoing background art, since image processing is evenly performed on the whole of digital image information or on selected image data, image quality may be improved and partly degraded. Namely, when a smoothing filter is used to minimize a noise, image data representing the same structure has a noise component thereof minimized but the boundary between structural image data items is blurred. This results in an unclear image. When a sharpening filter is used, although the sharpening filer works reversely to the smoothing filter does, the same phenomenon takes place.

requested to be as fine as possible for the purpose of interpretation. Degradation in image quality is not preferred even if it is partial degradation. Moreover, in imaging modalities including X-ray CT systems, image quality can be upgraded by modifying the imaging conditions to the same extent as it is improved through image processing. For example, in the case of the X-ray CT system, image quality represented by tomographic image information can be improved by increasing a dose of X-rays irradiated to a subject. However, this increases a load on the subject and is therefore not preferable.

Consequently, what counts is how to realize an image processing method, an image processing system, and an X-ray CT system capable of producing image information that contains a plurality of structural image data items and that represents an image whose quality is even partly not degraded.

Therefore, an object of the present invention is to provide an image processing method, an image processing system, and an X-ray CT system capable of producing image information that contains a plurality of structural image data items and that represents an image whose quality is even partly not degraded.

In order to solve the foregoing problems and accomplish the above object, an image processing method in accordance with the first aspect of the present invention comprises the steps of: producing a graph of a cumulative distribution function that provides a cumulative number of pixels, which is the number of pixels that are contained in digital image information comprising a plurality of pixels but do not exceed a certain pixel value, for each pixel value; working out a local standard deviation, which is a standard deviation of values of local pixels belong to an investigation domain comprising a predefined number of pixels determined with the cumulative numbers of pixels, using the graph of the cumulative distribution function, and repeating the working-out while shifting the investigation domain so that the investigation domain will cover all the cumulative numbers of pixels; detecting the smallest value among the plurality of local standard deviations resulting from the repetition, and multiplying the smallest value by a plurality of region designation values so as to calculate a plurality of region identification thresholds; classifying the plurality of local standard deviations on the basis of the plurality of domain identification thresholds; selecting an image processing parameter for each of categories; and performing image processing on image data, which is contained in the digital image information and of which local standard deviation is designated with the category, using the image processing parameter.

According to the first aspect of the present invention, a graph of a cumulative distribution function is produced from digital image information. The graph of the cumulative distribution function is used to repeatedly calculate a local standard deviation. Region identification thresholds are calculated based on the smallest value among the plurality of local standard deviations. The local standard deviations are classified based on the region identification thresholds. An image processing parameter is selected for each of categories. The selected image processing parameter is used to perform image processing on image data which is contained in the digital image information and of which local standard deviation is designated with the category.

An image processing method in accordance with the second aspect of the present invention is characterized in that the image processing parameter includes weight coefficients defining a smoothing filter which smoothes pixel values.

According to the second aspect of the present invention, the image processing parameter includes weight coefficients defining a smoothing filter.

An image processing method in accordance with the third aspect of the present invention is characterized in that the working-out is to work out an overall standard deviation that is a local standard deviation of a predefined number of pixels equivalent to a sum total of pixels contained in the digital image information.

According to the third aspect of the present invention, the magnitude of the dispersion of pixels contained in each piece of digital image information is evaluated based on the overall standard deviation.

Moreover, an image processing method in accordance with the fourth aspect of the present invention is characterized in that the calculation is to calculate a boundary identification threshold on the basis of the overall standard deviation.

According to the fourth aspect of the present invention, the boundary identification threshold is set to the same value relative to all pieces of digital image information.

Moreover, an image processing method in accordance with the fifth aspect of the present invention is characterized in that the image processing parameter include weight coefficients defining a sharpening filter that sharpens pixel values.

According to the fifth aspect of the present invention, the image processing parameter includes weight coefficients defining the sharpening filter designed for sharpening.

Moreover, an image processing method in accordance with the sixth aspect of the present invention is characterized in that the selection is to select based on the boundary identification threshold whether the image processing parameter for each category defines a smoothing filter or a sharpening filter or defines no change in pixel values.

According to the sixth aspect of the present invention, the selection is to determine whether the smoothing filter or the sharpening filter is adopted or pixel values are not changed.

Moreover, an image processing method in accordance with the seventh aspect of the present invention is characterized in that when the selection is to, when an image processing parameter defining the sharpening filter is selected, designate the weight parameters for a category of a local standard deviation exceeding the largest value among the plurality of region identification thresholds.

According to the seventh aspect of the present invention, the selection is to select the sharpening filter for the category of the largest local standard deviation.

Moreover, an image processing method in accordance with the eighth aspect of the present invention is characterized in that the weight coefficients are normalized by the sum total of all weight coefficients specified in a kernel of the smoothing filter or sharpening filter.

According to the eighth aspect of the present invention, the weight coefficients are normalized in order to confine pixel values to a predetermined range.

Moreover, an image processing method in accordance with the ninth aspect of the present invention is characterized in that the pixel value is represented by a CT number adapted to digital image information produced by an X-ray CT system.

According to the ninth aspect of the present invention, digital image information produced by an X-ray CT system is employed.

Moreover, an image processing system in accordance with the tenth aspect of the present invention comprises: a producing means for producing a graph of a cumulative distribution function that provides a cumulative number of pixels, which is the number of pixels that are contained in digital image information comprising a plurality of pixels and that do not exceed a certain pixel value, for each pixel value; a working-out means for working out a local standard deviation that is a standard deviation of values of local pixels belonging to an investigation domain including a predefined number of pixels determined with the cumulative numbers of pixels, using the cumulative distribution function, and repeating the working-out while shifting the investigation domain so that the investigation domain will cover all the cumulative numbers of pixels; a calculating means for detecting the smallest value among the plurality of local standard deviations resulting from the working-out, and multiplying the smallest value by a plurality of region designation values so as to calculate a plurality of region identification thresholds; a classifying means for classifying the plurality of local standard deviations on the basis of the plurality of region identification thresholds; a selecting means for selecting an image processing parameter for each of categories; and a processing means for using the image processing parameter to performing image processing on image data which is contained in the digital image information and whose local standard deviation is designated with the category.

According to the tenth aspect of the present invention, the producing means produces a graph of a cumulative distribution function from digital image information. The working-out means uses the graph of the cumulative distribution function to repeatedly work out a local standard deviation. The calculating means calculates region identification thresholds on the basis of the smallest value among the plurality of local standard deviations. The classifying means classifies the local standard deviations on the basis of the region identification thresholds. The selecting means selects an image processing parameter for each category. The processing means uses the selected image processing parameter to perform image processing on image data which is contained in the digital image information and whose local standard deviation is designated with the category.

Moreover, an image processing system in accordance with the eleventh aspect of the present invention is characterized in that the image processing parameter refers to weight coefficients that define a smoothing filter which smoothes pixel values.

According to the eleventh aspect of the present invention, the image processing parameter includes the weight coefficients that define to the smoothing filter.

Moreover, an image processing system in accordance with the twelfth aspect of the present invention is characterized in that the working-out means works out an overall standard deviation that is a local standard deviation of a predefined number of pixels equivalent to a total number of pixels contained in the digital image information.

According to the twelfth aspect of the present invention, the working-out means works out the overall standard deviation of all pixels contained in digital image information.

Moreover, an image processing system in accordance with the thirteenth aspect of the present invention is characterized in that the calculating means calculates a boundary identification threshold on the basis of the overall standard deviation and the smallest value.

According to the thirteenth aspect of the present invention, the calculating means calculates the boundary identification threshold on the basis of the overall standard deviation and the smallest value.

Moreover, an image processing system in accordance with the fourteenth aspect of the present invention is characterized in that the image processing parameter refers to weight coefficients defining a sharpening filter that sharpens pixel values.

According to the fourteenth aspect of the present invention, the image processing parameter includes the weight coefficients defining the sharpening filter that sharpens pixel values.

An image processing system in accordance with the fifteenth aspect of the present invention is characterized in that the selecting means selects based on the boundary identification threshold whether the image processing parameter for the category defines the smoothing filter or the sharpening filter or defines no change in pixel values.

According to the fifteenth aspect of the present invention, the selecting means selects whether the smoothing filter or sharpening filter is adopted or whether pixel values are not changed.

Moreover, an image processing system in accordance with the sixteenth aspect of the present invention is characterized in that the when selecting means selects an image processing parameter, which defines the sharpening filter, as the image processing parameter for each category, the selecting means designates the weight coefficients for a category of a local standard deviation exceeding the largest value among the plurality of region identification thresholds.

According to the sixteenth aspect of the present invention, the selecting means selects the sharpening filter relative for the category of the largest local standard deviation.

Moreover, an image processing system in accordance with the seventeenth aspect of the present invention is characterized in that the weight coefficients are normalized by the sum total of all weight coefficients specified in the kernel of the smoothing filter or sharpening filter.

According to the seventeenth aspect of the present invention, the weight coefficients are normalized in order to confine pixel values to a predetermined range.

Moreover, an image processing system in accordance with the eighteenth aspect is characterized in that the pixel value is represented by a CT number adapted to digital image information produced by an X-ray CT system.

According to the eighteenth aspect of the present invention, digital image information produced by an X-ray CT system is employed.

Moreover, an X-ray CT system in accordance with the nineteenth aspect of the present invention comprises a scanner gantry that irradiates an X-ray beam to a subject and acquires projection data from the subject, and a scanner console that produces digital image information on the subject through image reconstruction performed on the projection data. The scanner console includes an image processing system comprising: a producing means for producing a graph of a cumulative distribution function that provides a cumulative number of pixels, which is the number of pixels contained in digital image information and that do not exceed a certain pixel value, for each pixel value; a working-out means for working out a local standard deviation, which is a standard deviation of values of local pixels belonging to an investigation domain including a predefined number of pixels determined with the cumulative numbers of pixels, using the cumulative distribution function, and repeating the working-out while shifting the investigation domain so that the investigation domain will cover all the cumulative numbers of pixels; a calculating means for detecting the smallest value among a plurality of local standard deviations provided resulting from the working-out, and multiplying the smallest value by a plurality of region designation values so as to calculate a plurality of region identification thresholds; a classifying means for classifying the plurality of local standard deviations on the basis of the plurality of region identification thresholds; a selecting means for selecting an image processing parameter for each of categories; and a processing means for using the image processing parameter to performing image processing on image data which is contained in the digital image information and whose local standard deviation is designated with the category.

According to the nineteenth aspect of the present invention, in the image processing system included in the scanner console, the producing means produces a graph of a cumulative distribution function from digital image information. The working-out means uses the graph of the cumulative distribution function to repeatedly work out a local standard deviation. The calculating means calculates region identification thresholds according to the smallest value among the plurality of local standard deviations. The classifying means classifies local standard deviations on the basis of the region identification thresholds. The selecting means selects an image processing parameter for each category. The processing means uses the selected image processing parameter to perform image processing on image data which is calculated in the digital image information and of which local standard deviation is designated with the category.

As described above, according to the present invention, a graph of a cumulative distribution function is produced from digital image information. The graph of the cumulative distribution function is used to repeatedly work out a local standard deviation. Region identification thresholds are calculated based on the smallest value among the plurality of local standard deviations. The local standard deviations are classified based on the region identification thresholds. An image processing parameter is selected for each of categories. The selected image processing parameter is used to perform image processing on image data which is contained in the digital image information and whose local standard deviation is designated with the category. Consequently, a plurality of structural image data items contained in digital image information is sampled, and an optimal image processing parameter is used to perform image processing on each structural image data or each boundary between structural image data items. Therefore, partial degradation in image quality caused by image processing performed using the same image processing parameter can be prevented, and partial degradation in image quality that is manifested as a streaky artifact or the like can be alleviated without entire degradation of image quality.

Further objects and advantages of the present invention will be apparent from the following description of the preferred embodiments of the invention as illustrated in the accompanying drawings, in which:

Fig. 1 is a block diagram showing the overall configurations of an image processing system and an X-ray CT system.

Fig. 2 is a functional block diagram showing the functional configuration of the image processing system.

Fig. 3 shows tomographic image information and a region of processing.

Fig. 4 shows a graph of a distribution function of a pixel value and a graph of a cumulative distribution function.

Fig. 5 shows investigation domains indicated in the graph of the cumulative distribution function and local standard deviations worked out from the graph.

Fig. 6 shows an example of a kernel.

Fig. 7 shows examples of kernels associated with categories.

Fig. 8 is a flowchart describing actions to be performed in the image processing system in accordance with the first embodiment.

Fig. 9 shows a graph of a cumulative distribution function produced in a case where no structural image data is contained in a region of processing.

Fig. 10 is a flowchart (part 1) describing actions to be performed in an image processing system in accordance with the second embodiment.

Fig. 11 is a flowchart (part 2) describing the actions to be performed by the image processing system in accordance with the second embodiment.

Referring to accompanying drawings, an image processing method, an image processing system, and an X-ray CT system in accordance with preferred embodiments of the present invention will be described below.

(First Embodiment)

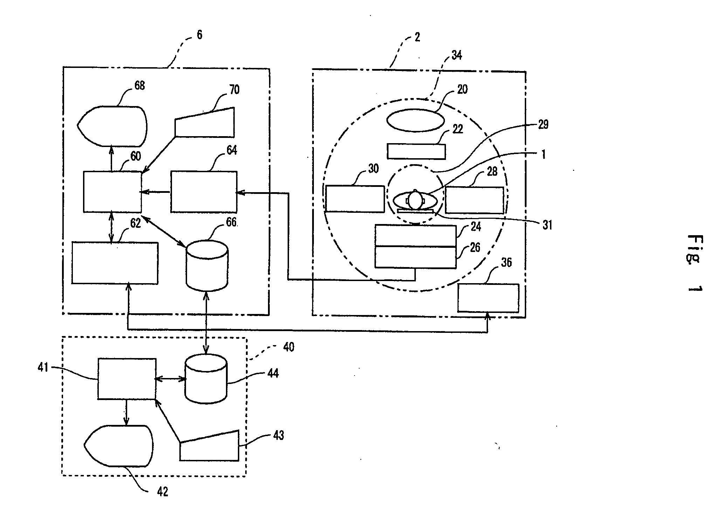

To begin with, the overall configurations of an image processing system and an X-ray CT system in accordance with the first embodiment will be described below. Fig. 1 is a block diagram of the X-ray CT system. As shown in Fig. 1, the X-ray CT system includes a scanner gantry 2 and an operator console 6.

The scanner gantry 2 includes an X-ray tube 20. X-rays that are not shown and are radiated from the X-ray tube 20 are recomposed into, for example, a fan-shaped X-ray beam or so-called fan-beam X-rays by a collimator 22, and irradiated to a detector array 24.

The detector array 24 has a plurality of X-ray detector elements set in array in a direction in which the fan-beam X-rays spread. The detector array 24 is a multi-channel X-ray detector having the plurality of X-ray detector elements set in array.

The plurality of detector array 24 forms a cylindrically concave X-ray incidence surface as a whole. Each of the detector array 24 comprises a combination of, for example, a scintillator and a photodiode, but is not limited to the combination. Alternatively, for example, a semiconductor X-ray detector element utilizing cadmium telluride (CdTe) or the like or an ion chamber type X-ray detector element employing a xenon gas will do. The X-ray tube 20, collimator 22, and detector array 24 constitute an X-irradiation/detection assembly.

A data acquisition unit 26 is connected to the detector array 24. The data acquisition unit 26 acquires detection data detected by each of the X-ray detector elements constituting the detector array 24. X-irradiation by the X-ray tube 20 is controlled by an X-ray controller 28. The illustration of the connective relationship between the X-ray tube 20 and X-ray controller 28 and the connective relationship between the collimator 22 and a collimator controller 30 will be omitted. The collimator 22 is controlled by the collimator controller 30.

The foregoing components starting with the X-ray tube 20 and ending with the collimator controller 30 are incorporated in a rotary section 34 of the scanner gantry 2. Herein, a subject 1 is asked to lie down on a cradle 31 on his/her side and carried into a bore 29 in the center of the rotary section 34. The rotary section 34 rotates under the control of a rotation controller 36. The X-ray tube 20 shoots X-rays and the detector array 24 detects X-rays transmitted by the subject 1. The illustration of the connective relationship between the rotary section 34 and rotation controller 36 will be omitted.

The operator console 6 includes an image reconstruction unit 60. The image reconstruction unit 60 is realized with, for example, a computer. A control interface 62 is connected to the image reconstruction unit 60. The scanner gantry 2 is connected to the control interface 62. The image reconstruction unit 60 controls the scanner gantry 2 via the control interface 62.

The data acquisition unit 26, X-ray controller 28, collimator controller 30, and rotation controller 36 that are incorporated in the scanner gantry 2 are controlled via the control interface 62. The illustration of the connections of the control interface 62 to the data acquisition unit 26, X-ray controller 28, collimator controller 30, and rotation controller 36 will be omitted.

A data collection buffer 64 is connected to the image reconstruction unit 60. The data acquisition unit 26 incorporated in the scanner gantry 2 is connected to the data collection buffer 64. Data acquired by the data acquisition unit 26 is transferred to the image reconstruction unit 60 via the data collection buffer 64.

The image reconstruction unit 60 reconstructs an image using a transmitted X-ray signal, that is, projection data acquired via the data collection buffer 64. Moreover, a storage unit 66 is connected to the image reconstruction unit 60. Projection data collected into the data collection buffer 64, tomographic image information representing a reconstructed image, and programs or the like for implementing the facilities of the system are stored in the storage unit 66.

Moreover, a display device 68 and an operating device 70 are connected to the image reconstruction unit 60. Tomographic image information and other information provided by the image reconstruction unit 60 are displayed on the display device 68. An operator manipulates the operating device 70 so as to enter various instructions or various pieces of information that are transferred to the image reconstruction unit 60. The operator uses the display device 68 and operating device 70 to operate the X-ray CT system interactively.

An image processing system 40 is connected to the storage unit 66 incorporated in the operator console 6 via a storage unit 44 by means of a communicating means that is not shown. The image processing system 40 is disposed on a console independent of the operator console 6. The image processing system transfers tomographic image information, which represents a reconstructed image and is stored in the storage unit 66, to the storage unit 44, and an image processing unit 41 performs image processing. The image processing unit 41 is realized with, for example, a computer.

Moreover, a display device 42 and an operating device 43 are connected to the image processing unit 41. Tomographic image information and other information sent from the image processing unit 41 are displayed on the display device 42. An operator manipulates the operating device 43 so as to enter various instructions or various pieces of information that are transferred to the image processing unit 41. The operator uses the display device 42 and operating device 43 to interactively operate the image processing system.

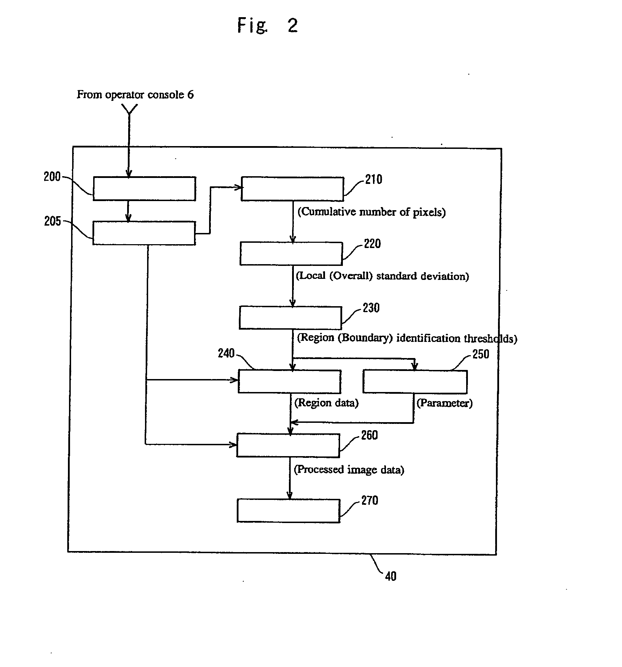

Fig. 2 is a functional block diagram of the image processing system 40. The image processing system 40 includes, in addition to tomographic image information 200, a region delineating means 205, a designating/producing means 210, a working-out means 220, a calculating means 230, a classifying means 240, a selecting means 250, a processing means 260, and a display means 270.

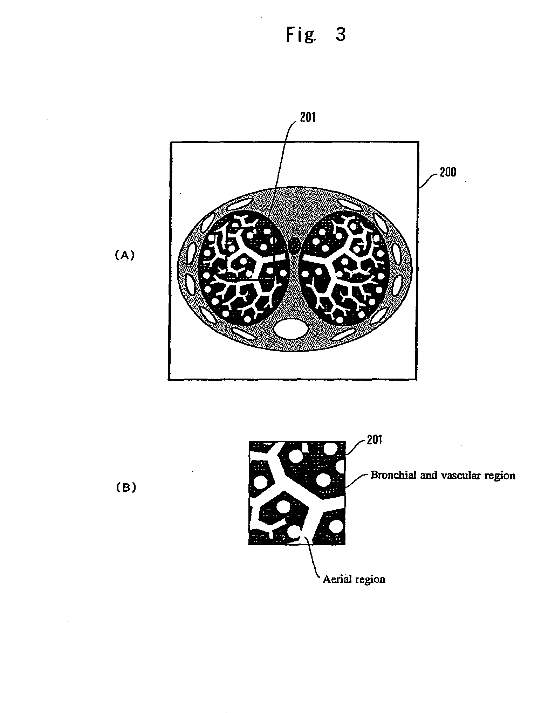

The tomographic image information 200 is digital image information that represents a tomographic image of an axial section of the subject 1 and that is transferred from the storage unit 66 incorporated in the operator console 6 to the storage unit 44. Fig. 3(A) shows an example of the tomographic image information 200 representing an image of the lung fields of the subject 1. Fig. 3(A) shows the whole of the tomographic image information 200 comprising, for example, a matrix of 512 pixels in rows and 512 pixels in columns. Each pixel assumes a value indicating a shade in a 256-level gray scale. In the case of the X-ray CT system, the pixel value is represented by a CT number standardized by defining a pixel value obtained from water as 0 and a pixel value obtained from air as -1000.

The region delineating means 205 delineates a region of processing 201 so as to designate an object of image processing to be described later. During the delineation, a region of interest (ROI) is delineated in a tomographic image displayed on the display device 42, and image processing is performed on pixels within the region. The region of interest is, as shown in Fig. 3(A), for example, a rectangular region whose position in a tomographic image and whose size are selected by an input through the operating device 43.

Fig. 3(B) shows tomographic image information 200 sampled from the region of processing 201 shown in Fig. 3(A). Referring to Fig. 3(B), the tomographic image information 200 containing image data items that represent an image of the bronchia and blood vessels and an image of the air in the bronchia is sampled from the tomographic image of the entire lung fields shown in Fig. 3(A). Hereinafter, a description will proceed on the assumption that image processing is performed on the tomographic image information 200 alone sampled from the region of processing 201. When a region on which image processing will be performed is delineated as the region of processing 201, the image processing time can be shortened and the image processing can be achieved more effectively.

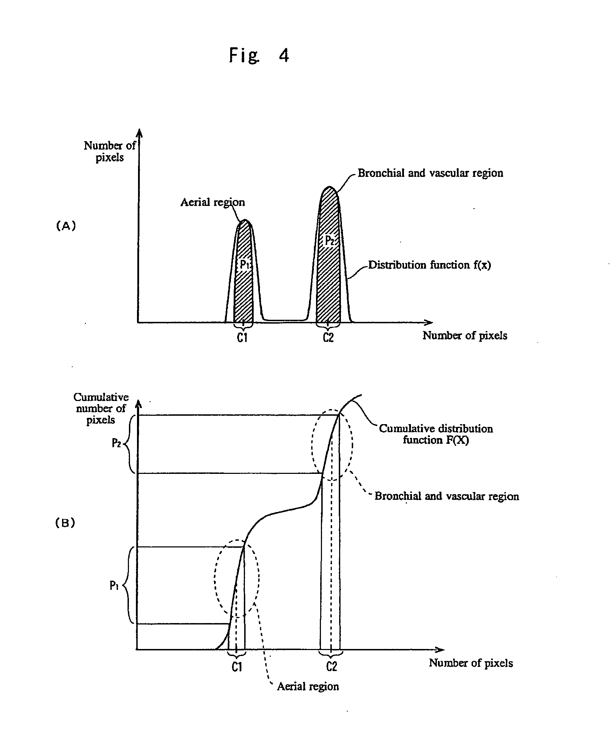

The producing means 210 produces a graph of a cumulative distribution function that provides the number of pixels, which assume a certain pixel value, in relation to each pixel value. The cumulative distribution function will be described in conjunction with Fig. 4 by taking the tomographic image information 200 shown in Fig. 3(B) for instance. The tomographic image information 200 shown in Fig. 3(B) contains mainly two image data items contained in the bronchial and vascular region and in the aerial region in the bronchial region. In each of the two regions, generally all pixels other than a noise component assume the same pixel value. Therefore, assuming that the axis of abscissas indicates a pixel value and the axis of ordinates indicates the number of pixels, a graph of a distribution function f(x) indicating the number of pixels that assumes a maximum value in relation to a pixel value contained in the bronchial and vascular region and a pixel value contained in the aerial region respectively is plotted as shown in Fig. 4(A).

Now, a cumulative distribution function providing a cumulative number of pixels that is a total number of pixels that do not exceed a certain pixel value X and that are contained in the region of processing 201 shall be F(X). At this time, a graph of the function F(X) is plotted as shown in Fig. 4(B). Mathematically, the functions f(x) and F(X) have a relationship expressed as follows:

[Mathematical]

$$F(X) = \int_{-1000}^{X} f(x)dx$$

The lower limit of the integration is set to the smallest pixel value. Namely, when the pixel value is represented by a CT number, the lower limit of the integration is set to the CT number of -1000 obtained from air. The relationship between the functions f(x) and F(X) is identical to the relationship between a probability density function and a cumulative distribution function that are defined in the theory of statistical probability, and the mathematical natures thereof are conformable to those of the probability density function and cumulative distribution function.

Assume that, based on the foregoing definitions, the total number of pixels that belongs to a domain C1 of pixel values constituting the aerial image data which is indicated in the graph of the distribution function f(x) shown in Fig. 4(A) is P1, and the total number of pixels that belongs to a domain C2 of pixel values constituting the bronchial and vascular image data is P2. In this case, a change in the cumulative number of pixels occurring in relation to the domain C1 of pixel values indicated in the graph of the cumulative distribution function F(X) shown in Fig. 4(B) is P1, and a change in the cumulative number of pixels occurring in relation to the domain C2 of pixel values is P2. Consequently, the structural image data items contained in the region of processing 201, for example, the image data items contained in the bronchial and vascular region and the aerial region are regarded as image data items having a large total number of pixels P1 or P2, and expressed as portions of the graph of the cumulative distribution function F(X) having sharp gradients as shown in Fig. 4(B). Reversely, when the portions of the graph of the cumulative distribution function shown in Fig. 4(B) which have sharp gradients are sampled, the structural image data items contained in the region of processing 201 can be sampled and the pixel values constituting the structural image data items can be detected.

Incidentally, pixel values contained in actual tomographic image information 200 and the numbers of pixels specified therein are digital values. In this case, the cumulative distribution function F(X) is calculated by adding the number of pixels assuming the same pixel value to a cumulative number of pixels indicated by the axis of ordinates while sequentially increasing the pixel value from the smallest value of, for example, -1000. The initial value on the axis of ordinates is set to zero.

The working-out means 200 works out a local standard deviation of pixel values using the graph of the cumulative distribution function F(X) produced by the producing means 210. The graph of the cumulative distribution function F(X) produced by the producing means 210 encompasses the structural image data and noise data that are contained in the region of processing 201. Consequently, the pixel values contained in the tomographic image information 200 exhibit dispersion about a true value. In order to sample the pixels that are indicated with the portion of the graph having a sharp gradient as shown in Fig. 4(B) and that exhibit dispersion, a local standard deviation is calculated. Assuming that the number of samples that are pixel values is N, an individual pixel value is c, and a mean of pixel values or samples is μ, a standard deviation σ is defined as follows:

$$\sigma^2 = \Sigma(c-\mu)^2 / N$$

The standard deviation indicates the magnitude of the dispersion of the samples about the mean. When the samples are limited to pixel values belonging to an investigation domain that will be described later, the standard deviation of the samples is regarded as a local standard deviation.

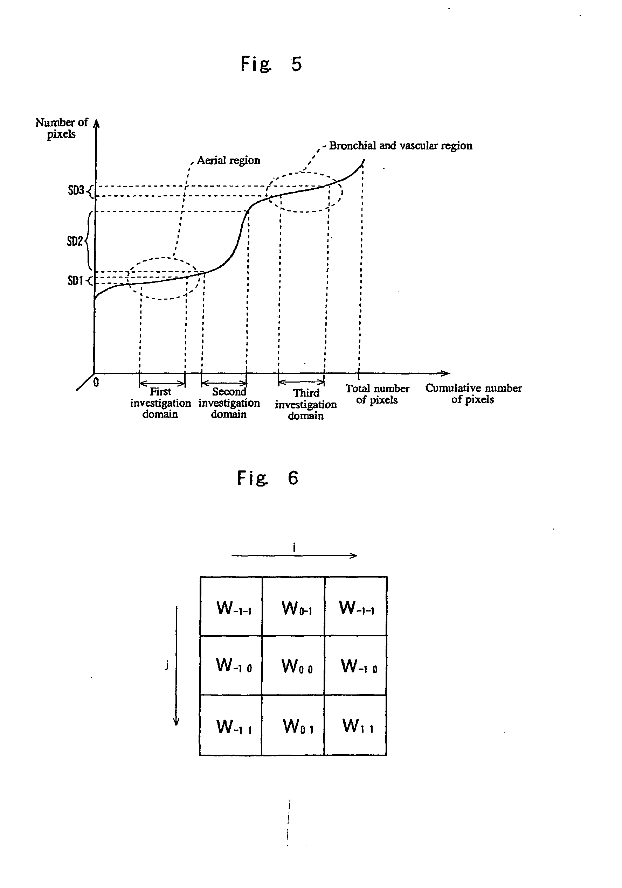

Fig. 5 illustratively shows a way of calculating a local standard deviation using the graph of the cumulative distribution function F(X). Referring to Fig. 5, the axis of abscissas indicates a cumulative number of pixels and the axis of ordinates indicates a pixel value. What are indicated by the axes of abscissas and ordinates are reverse to those indicated thereby shown in Fig. 4(B). This is intended to clearly show a process of calculating a local standard deviation of pixel values in relation to the cumulative number of pixels. The portions of the graph having the sharp gradients and indicating the pixels that constitute the bronchial and vascular region and the aerial region respectively correspond to even portions of the graph of Fig. 5. The even portions are sampled using the local standard deviation. Incidentally, each of domains having predetermined widths on the axis of abscissas shown in Fig. 5 signifies the number of pixels. Therefore, a domain having a double width signifies a double number of pixels.

The working-out means 220 designates an investigation domain composed of a predefined number of pixels on the axis of abscissas indicating the cumulative number of pixels. The predefined number of pixels is designated using the operating device 43 included in the image processing system 40. An operator designates the predefined number of pixels in consideration of the number of pixels contained in the region of processing 20 or the size of structural image data contained in the region of processing 201. The working-out means 220 then works out a local standard deviation that is a standard deviation of pixel values determined with cumulative numbers of pixels at the ends of the investigation domain.

Fig. 5 shows local standard deviations SD1 to SD3 calculated from the first to third investigation domains. The first investigation domain signifies the pixels constituting the aerial region. Since the portion of the graph of the cumulative distribution function F(X) relevant to the first investigation domain is even, the pixels including noises exhibit a small local standard deviation SD1. The second investigation domain signifies the pixels constituting a boundary region. Since the portion of the graph of the cumulative distribution function F(X) relevant to the second investigation domain has a sharp gradient, the pixels including noises exhibit a large local standard deviation SD2. The third investigation domain signifies the pixels constituting the bronchial and vascular region. Since the portion of the graph of the cumulative distribution function F(X) relevant to the third investigation domain is even, the pixels including noises exhibit a small local standard deviation SD3. Namely, the local standard deviation SD2 is larger than the local standard deviations SD1 and SD3. The investigation domains exhibiting the small local standard deviations signify the structural image data items.

The cumulative numbers of pixels determining the investigation domain are changed every time a local standard deviation is calculated. This is repeated until the local standard deviation is calculated relative to all the cumulative numbers of pixels. The cumulative numbers of pixels are changed in units of a predefined number of pixels included in the investigation domain, whereby a failure to sample structural image data can be avoided and all cumulative numbers of pixels can be taken into account. Fig. 5 shows only the first to third investigation domains as typical examples exhibiting largely different local standard deviations.

The calculating means 230 detects the smallest standard deviation that is the smallest value among the local standard deviations worked out by the working-out means 220, and then calculates region identification thresholds on the basis of the smallest standard deviation. Herein, the region identification threshold is a threshold serving as a criterion based on which structural image data such as image data contained in the bronchial and vascular region or the aerial region is sampled. Moreover, the calculating means 230 multiplies the smallest standard deviation by a plurality of region designation values. The region designation values are entered at the operating device 43 included in the image processing system 40. An operator designates the region designation values and the number of region designation values. For example, when the region designation values are set to 1 and 2, the calculated region identification thresholds correspond to local standard deviations calculated as products of the smallest standard deviation by 1 and 2 respectively. Hereinafter, a description will be made of a case where two region designation values are designated for brevity's sake. However, the number of region designation values is not limited to 2 but may be 3 or larger.

The classifying means 240 classifies pixel values on the basis of the region identification thresholds calculated by the calculating means 230. For example, when the region identification thresholds are the products of the smallest standard deviation by 1 and 2 respectively, pixel values exhibiting a local standard deviation equal to or smaller than the product of the smallest standard deviation by 1 are classified into a category A. Pixel values exhibiting a local standard deviation larger than the product of the smallest standard deviation by 1 and equal to or smaller than the product of the smallest standard deviation by 2 are classified into a category B. Pixel values exhibiting a local standard deviation larger than the product of the smallest standard deviation by 2 are classified into a category C. Thus, all the pixel values are classified into any of the categories A to C.

The selecting means 250 designates an image processing parameter for each category. Herein, weight coefficients that define a smoothing filter or a sharpening filter which performs spatial filtering or that define no change in pixel values is adopted as the image processing parameter. Now, the spatial filtering that is image processing to be performed on the tomographic image information 200 will be described below.

For the spatial filtering, an arithmetic and logic operation is performed on values of pixels neighboring each of the pixels, which are contained in the tomographic image information 200, with the pixel as a center in order to work out a new pixel value. A convolution kernel (hereinafter a kernel) of a spatial filter is applied to the neighboring pixels on which the arithmetic and logic operation is performed. Fig. 6 shows an example of the kernel. The kernel has a matrix of three pixels in rows and in columns. Each of the pixel locations in the matrix is represented by a parameter i indicating a sideways position and a parameter j indicating a lengthwise position. Moreover, a weight coefficient $W_{ij}$ is allocated to each pixel location. A pixel value B in the center of the matrix is provided by assigning pixel values $A_{ij}$ at respective pixel locations to the following expression:

[Mathematical]

$$ B = \sum_i \sum_j Wij\ Aij $$

A spatial filter is defined as a smoothing filter or a sharpening filter according to the values of the weight coefficients $W_{ij}$. For example, when the weight coefficients $W_{ij}$ allocated to respective pixel locations are all 1 s, the spatial filter is defined as a smoothing filter that adopts an average as a new pixel value. When the weight coefficient $W_{ij}$ allocated to the center pixel location, that is, a weight coefficient $W_{00}$ assumes a negative value and the other weight coefficients assume positive values, the spatial filter is defined as a sharpening filter.

The pixel value B is normalized by the sum total W of the weight coefficients $W_{ij}$ expressed below.

[Mathematical]

$$ W = \sum_i \sum_j Wij $$

A pixel value B'=B/W is a new pixel value having undergone image processing. Thus, pixel values can be confined to a predetermined range.

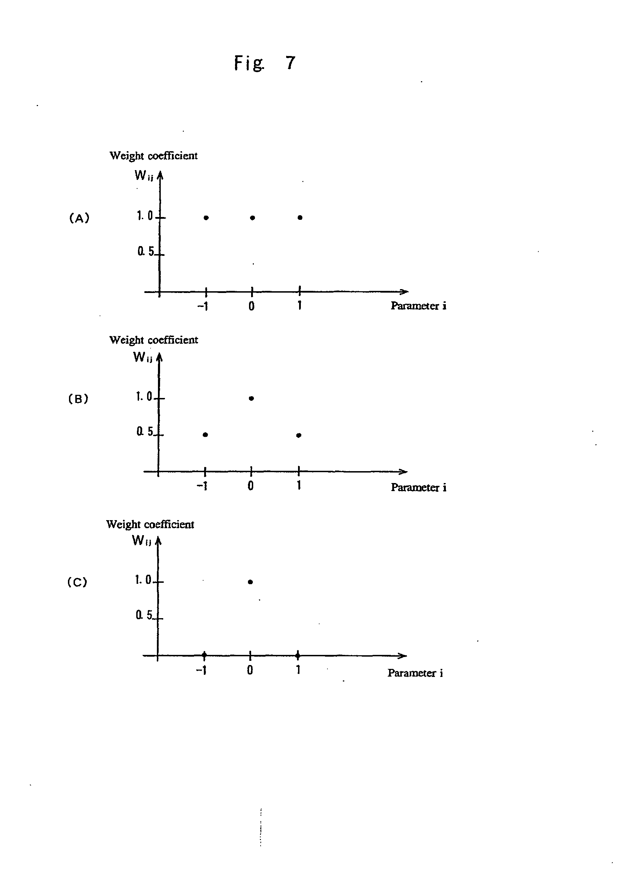

The selecting means 250 selects optimal weight coefficients $W_{ij}$ for each category. Fig. 7 shows examples of the weight coefficients $W_{ij}$ to be selected relative to the categories A to C as the weight coefficients to be specified in the kernel shown in Fig. 6. The same as those shown in Fig. 7 applies to the weight coefficients associated with the parameters j specified in the kernel shown in Fig. 6. Fig. 7(A) shows the kernel that intensively performs smoothing and has all the weight coefficients $W_{ij}$ set to 1. The weight coefficients $W_{ij}$ are associated with the category A to which pixels in the center of structural image data belong. Fig. 7(B) shows the kernel that little performs smoothing and has the center weight coefficient $W_{00}$ set to 1 and the other weight coefficients $W_{ij}$ set to 0.5. The weight coefficients $W_{ij}$ are associated with the category B to which pixels interposed between the center and margin of structural image data belong. Fig. 7(C) shows the kernel that does not perform smoothing and has the center weight coefficient $W_{00}$ set to 1 and the other weight coefficients $W_{ij}$ set to 0. The weight coefficients $W_{ij}$ are associated with the category C to which pixels on the margin of structural image data belong. Consequently, when the kernel shown in Fig. 7(C) is selected, image processing is not performed at all, and pixel values are therefore not changed at all.

The processing means 260 performs image processing according to the category into which pixels are classified by the classifying means 240 and the weight coefficients $W_{ij}$ selected for the category by the selecting means 250. Herein, the processing means 260 selects a category on the basis of the values of pixels, which constitute image data, for each image data contained in the region of processing 201, and applies a spatial filter in which the weight coefficients $W_{ij}$ associated with the category are specified. For example, assume that the region identification thresholds include two thresholds that are the products of the smallest standard deviation by 1 and 2 respectively, and that all pixel values are classified into any of three categories A to C. In this case, if pixels to be subjected to image processing assume pixel values belonging to the category B, a spatial filter whose kernel specifies the weight coefficients $W_{ij}$ as shown in Fig. 7(B) is applied to the pixels.

The display means 270 displays processed image information on which image processing is performed by the processing means 260.

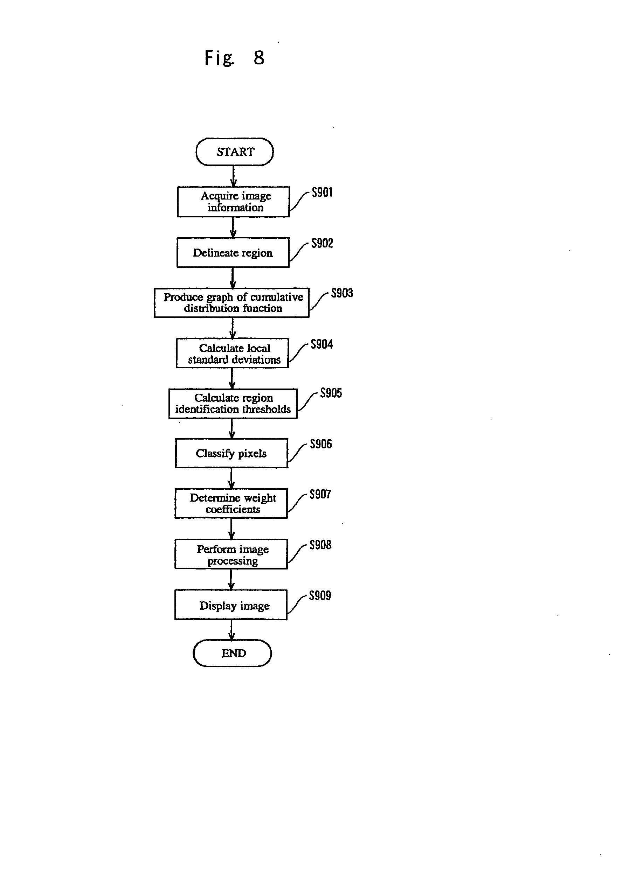

Next, actions to be performed in the image processing system 40 will be described in conjunction with Fig. 8. Fig. 8 is a flowchart describing the actions to be performed in the image processing system 40. First, an operator manipulates the operator console 6 to acquire tomographic image information 200 (step S901). The tomographic image information 200 is digital tomographic image information on the subject acquired by the scanner gantry 2. The operator then uses the region delineating means 205 to sample the region of processing 201 from the tomographic image information 200 (step S902).

Thereafter, the image processing system 40 uses the producing means 210 to calculate a cumulative number of pixels, which assume a certain pixel value, for each pixel value from the tomographic image information 200 contained in the region of processing 201. The producing means 200 then produces a graph of a cumulative distribution function F(X) (step S903). The image processing system 40 then uses the working-out means 220 to repeatedly work out a local standard deviation SD over the entire range of cumulative numbers of pixels indicated in the graph of the cumulative distribution function F(X) (step S904).

Thereafter, the image processing system 40 uses the calculating means 230 to obtain the smallest standard deviation among the local standard deviations and calculate region identification thresholds on the basis of region designation values designated by the operator (step S905). The image processing system 40 then uses the classifying means 240 to classify the pixels contained in the region of processing 201 on the basis of the local standard deviations according to the region identification thresholds (step S906).

Moreover, the image processing system 40 uses the selecting means 250 to select the weight coefficients $W_{ij}$, that is, the kernel for each of the categories into which pixels are classified based on the region identification thresholds (step S907). Thereafter, the image processing system 40 uses the processing means 260 to perform pixel by pixel image processing on the tomographic image information 200 contained in the region of processing 201 by applying the kernel selected for each category (step S908).

Thereafter, the image processing system 40 displays on the display device 42 a tomographic image according to the image data contained in the region of processing 201 resulting from the image processing (step S909). The processing is then terminated.

As mentioned above, according to the first embodiment, a graph of a cumulative distribution function providing a cumulative number of pixels, which assume a certain value, for each pixel value is produced based on the tomographic image information 200 contained in the region of processing 201. A local standard deviation of pixel values is worked out using the graph of the cumulative distribution function. A plurality of region identification thresholds is calculated based on the smallest standard deviation that is the smallest value among the local standard deviations. The local standard deviations, or eventually, the pixels exhibiting the local standard deviations are classified based on the region identification thresholds. Moreover, the weight coefficients $W_{ij}$ that define a smoothing filter are selected for each of the categories into which the pixels are classified based on the region identification thresholds. The weight coefficients $W_{ij}$ are used to smooth the values of pixels contained in the tomographic image information 200. Consequently, structural image data items exhibiting small local standard deviations can be sampled based on the smallest standard deviation among the local standard deviations of image data items contained in the region of processing 201, and smoothing can be performed on the structural image data items alone.

Moreover, according to the first embodiment, the image processing system 40 is connected to the operator console 6 over a communication line. Alternatively, the operator console 6 may be provided with the same capability as the capability of the image processing system 40.

Moreover, according to the first embodiment, tomographic image information produced by an X-ray CT system is processed. However, the present invention is not limited to the X-ray CT system. The present invention may be adapted to digital image information produced broadly by an X-ray imaging system, a magnetic resonant imaging system, or a nuclear medicine imaging system or to digital image information produced using a solid-state imaging device such as a CCD or a CMOS.

Moreover, according to the first embodiment, image processing is performed on two-dimensional tomographic image information. Alternatively, similar image processing may be performed on three-dimensional tomographic image information composed of a plurality of pieces of two-dimensional tomographic image information.

Moreover, according to the first embodiment, image processing is performed on static tomographic image information. Alternatively, similar image processing may be performed on digital motion picture information that varies time-sequentially.

Moreover, according to the first embodiment, different weight coefficients $W_{ij}$ that define a smoothing filter are selected for each of categories into which pixels are classified based on the region identification thresholds. The weight coefficients $W_{ij}$ are used to smooth pixels contained in the tomographic image information 200. Herein, the weight coefficients $W_{ij}$ may include weight coefficients, which do not change pixel values, like the ones shown in Fig. 7(C). The weight coefficients are applied especially to an edge of structural image data for the purpose of preventing blurring derived from smoothing.

(Second Embodiment)

According to the first embodiment, structural image data items contained in the region of processing 201 are sampled by calculating local standard deviations. Alternatively, boundary data between structural image data items contained in the region of processing 201 may be sampled according to the same method and sharpening may be performed. According to the second embodiment, the boundary data between structural image data items is sampled and sharpened.

The second embodiment of the present invention has the same hardware configuration as that shown in Fig. 1. The description of the hardware configuration will therefore be omitted. Moreover, the image processing system 40 shown in Fig. 2 includes a new facility in addition to the working-out means 220, calculating means 230, and selecting means 250. The added facility alone will be described below.

The selecting means 220 calculates an overall standard deviation that is a standard deviation of pixel values belonging to an investigation domain that includes a total number of pixels rather than a predefined number of pixels. The overall standard deviation is the measure of the overall dispersion of the tomographic image information 200 contained in the region of processing 201.

The calculating means 230 calculates a boundary identification threshold on the basis of the overall standard deviation worked out by the working-out means 220, and calculates a boundary region through comparison of the boundary identification threshold with the overall standard deviation. Incidentally, the boundary identification threshold is a threshold serving as a criterion for sampling pixels belonging to the second investigation domain 2 shown in Fig. 5 and being contained in the boundary region between the bronchial and vascular region and the aerial region.

Prior to a description of the boundary identification threshold, the boundary region between structural regions contained in the tomographic image information 200 will be described below. To begin with, the local standard deviation of pixels constituting the boundary region between structural regions is, as indicated with the example of the second investigation domain shown in Fig. 5, larger than the others. On the other hand, the local standard deviations of pixels constituting the tomographic image information 200 contained in the region of processing 201 greatly vary depending on structural image data items and imaging conditions. There is therefore difficulty in sampling a boundary region on the basis of a local standard deviation of a fixed value. The overall standard deviation is therefore adopted as a means for evaluating a difference between local standard deviations of pixels contained in each piece of tomographic image information 200. The calculating means 230 compares a ratio, which is calculated by dividing a local standard deviation by the overall standard deviation, with a boundary identification threshold. When the ratio exceeds the boundary identification threshold, a boundary region is identified. Consequently, the boundary region can be identified irrespective of a difference between local standard deviations of pixels contained in each piece of tomographic image information 200.

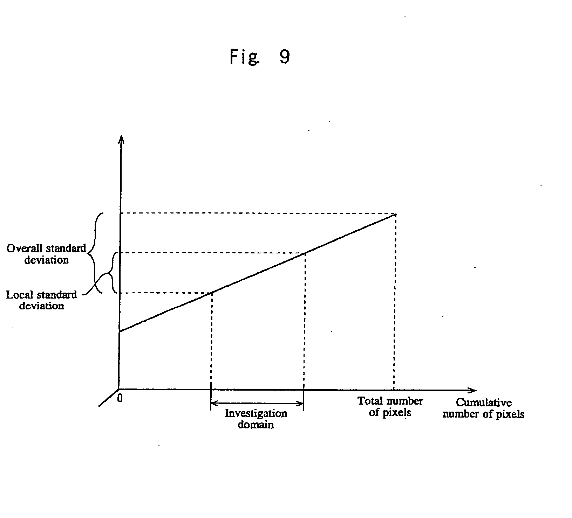

The local standard deviation varies depending on the width of an investigation domain including a predefined number of pixels and is therefore set to a predetermined value. For example, the predefined number of pixels is defined as a one-third of a total number of pixels contained in the region of processing 201, the boundary identification threshold is set to 1/3. The reason why the boundary identification threshold is set to 1/3 will be described in conjunction with Fig. 9. Fig. 9 shows a graph of a cumulative distribution function obtained when no specific structural image data is contained in the region of processing 201. In this case, the tomographic image information 200 comprises random noise components, and the pixel values are thought to be uniformly distributed within a predetermined width. In the graph of the cumulative distribution function shown in Fig. 9, pixel values are generally proportional to cumulative numbers of pixels. In this state, when the predefined number of pixels included in an investigation domain is a one-third of the total number of pixels, a local standard deviation is an approximately one-third of an overall standard deviation. When the ratio of a local standard deviation to the overall standard deviation exceeds 1/3, structural image data can be identified. Thus, the boundary identification threshold can be set to 1/3.

The selecting means 250 includes a kernel of a sharpening filter in which weight coefficients $W_{ij}$ to be selected for each category are specified. Herein, the weight coefficients $W_{ij}$ arranged in the form of a matrix as shown in Fig. 6 have the center weight coefficient $W_{00}$ and the other weight coefficients assigned different signs of positive and negative signs.

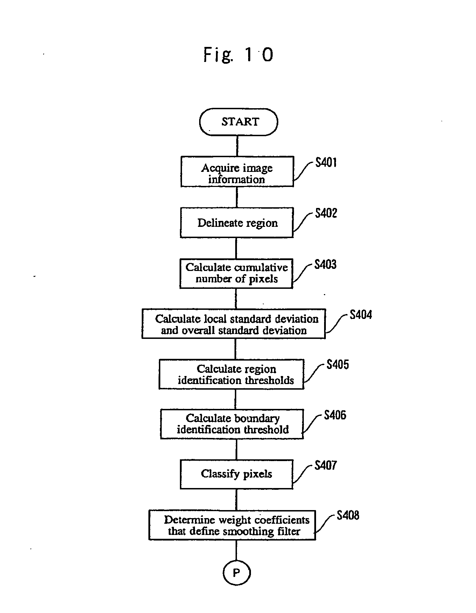

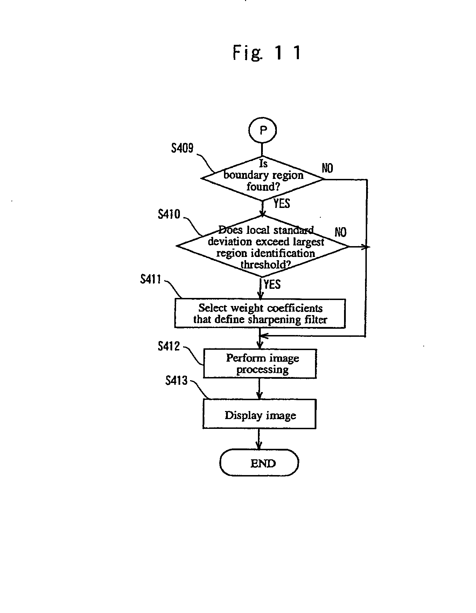

Actions to be performed in the image processing system 40 included in the second embodiment will be described in conjunction with Fig. 10 and Fig. 11. Fig. 10 and Fig. 11 are flowcharts describing the actions to be performed in the image processing system 40 included in the second embodiment. To begin with, an operator acquires tomographic image information 200 from the operator console 6 (step S401). The tomographic image information 200 is digital tomographic image information representing the subject 1 and being acquired by the scanner gantry 2. The operator uses the region delineating means 205 to delineate the region of processing 201 in the tomographic image information 200 (step S402).

Thereafter, the image processing unit 41 uses the producing means 210 to calculate a cumulative number of pixels, which assume a certain value, for each pixel value using the tomographic image information contained in the region of processing 201, and to produce a graph of a cumulative distribution function F (X) (step S403). The image processing unit 41 then uses the working-out means 220 to work out an overall standard deviation and also work out local standard deviations SD of pixels over the entire range of cumulative numbers of pixels (step S404). The investigation domain of pixels whose local standard deviation is worked out is set to an about one-third of a total number of pixels.

Thereafter, the image processing unit 41 uses the calculating means 230 to detect the smallest standard deviation among the local standard deviations and to calculate region identification thresholds using region designation values designated by an operator (step S405). Moreover, the image processing unit 41 uses the calculating means 230 to calculate a boundary identification threshold using the overall standard deviation (step S406). Herein, the boundary identification threshold is set to, for example, a value exceeding a one-third of the overall standard deviation. Moreover, the image processing unit 41 uses the classifying means 240 to classify the pixels, which are contained in the region of processing 201, by classify the local standard deviations of the pixels on the basis of the region identification thresholds (step S407). The image processing unit 41 then uses the selecting means 250 to select the weight coefficients $W_{ij}$, that is, the kernel of smoothing for each of categories into which the pixels are classified based on the region identification thresholds (step S408).

Thereafter, the image processing unit 41 uses the calculating means 230 to check pixels to see if the local standard deviations of the pixels exceed the boundary identification threshold so as to thus check whether a boundary region is identified (step S409). When a boundary region is identified (the checking is made in the affirmative at step S409), the local standard deviation of the pixels is checked to see if it exceeds the largest value among the region identification thresholds (step S410). When the local standard deviation exceeds the largest value among the region identification thresholds (the checking is made in the affirmative at step S410), the kernel of a sharpening filter is selected as the weight coefficients $W_{ij}$ for the category of pixels whose local standard deviation exceeds the largest value of the region identification thresholds (step S411).

Moreover, when pixels constituting a boundary region are not found (the checking is made in the negative at step S409) or although the pixels constituting a boundary region are found, the local standard deviation of the pixels does not exceed the largest value among the region identification thresholds (the checking is made in the negative at step S410), the image processing unit 41 does not select the sharpening filter at step S411 but processing proceeds to the next step.

Thereafter, the image processing unit 41 uses the processing means 260 to perform pixel by pixel image processing on the tomographic image information 200 contained in the region of processing 201 by applying the kernel associated with the category (step S412). The image processing unit 41 then displays a tomographic image represented by the image data, which is contained in the region of processing 201 and results from the image processing, on the display device 42 (step S413). The processing is then terminated.

As mentioned above, according to the second embodiment, a graph of a cumulative distribution function providing a cumulative number of pixels, which assume a certain value, for each pixel value is produced based on the tomographic image information 200 contained in the region of processing 201. The graph of the cumulative distribution function is used to work out local standard deviations of pixel values and an overall standard deviation. A plurality of region identification thresholds is calculated based on the smallest standard deviation that is the smallest value among the local standard deviations, and a boundary identification threshold is calculated based on the overall standard deviation. The local standard deviations, or eventually, the pixels exhibiting the local standard deviations are classified based on the region identification thresholds. Moreover, the weight coefficients $W_{ij}$ defining a smoothing filter are selected for each of categories into which the pixels are classified based on the region identification thresholds. Furthermore, if a boundary region composed of pixels whose local standard deviation exceeds the boundary identification threshold is found, the weight coefficients $W_{ij}$ defining a sharpening filter are selected for the boundary region. Thus, the weight coefficients $W_{ij}$ are applied to the pixels contained in the tomographic image information 200 for the purpose of smoothing or sharpening. Consequently, structural regions contained in the region of processing 201 and a boundary region between the structural regions can be sampled, and image processing, that is, smoothing and sharpening can be uniquely performed on each of the structural regions and boundary region.

Moreover, according to the second embodiment, the weight coefficients $W_{ij}$ defining a smoothing filter are uniquely selected for each of the categories into which pixels are classified based on the region identification thresholds. The weight coefficients $W_{ij}$ are applied to pixels contained in the tomographic image information 200 for the purpose of smoothing. The weight coefficients $W_{ij}$ may include weight coefficients defining no change in pixel values like the ones shown in Fig. 7 (C).

**Claims**

1. An image processing method comprising the steps of:

producing a graph of a cumulative distribution function that provides a cumulative number of pixels, which is the number of pixels that are contained in digital image information comprising a plurality of pixels and that do not exceed a certain pixel value, for each pixel value;
working out a local standard deviation, which is a standard deviation of values of local pixels belonging to an investigation domain that includes a predefined number of pixels determined with the cumulative numbers of pixels, using the graph of the cumulative distribution function, and repeating the working-out while shifting the investigation domain so that the investigation domain will cover all the cumulative numbers of pixels;
detecting the smallest value among the plurality of local standard deviations resulting from the repetition;
multiplying the smallest value by a plurality of region designation values so as to calculate a plurality of region identification thresholds;
classifying the plurality of local standard deviations on the basis of the plurality of region identification thresholds;

selecting an image processing parameter for each of categories; and performing image processing on image data, which is contained in the digital image information and of which local standard deviation is designated with the category, using the image processing parameter.

2. An image processing system (40) comprising:

a producing device (210) for producing a graph of a cumulative distribution function that provides a cumulative number of pixels, which is the number of pixels that are contained in digital image information comprising a plurality of pixels and that do not exceed a certain pixel value, for each pixel value;

a working-out device (220) for working out a local standard deviation, which is a standard deviation of values of local pixels belonging to an investigation domain including a predefined number of pixels determined with the cumulative numbers of pixels, using the graph of the cumulative distribution function, and repeating the working-out while shifting the investigation domain so that the investigation domain will cover all the cumulative numbers of pixels;

a calculating device (230) for detecting the smallest value among the plurality of local standard deviations resulting from the working-out, and multiplying the smallest value by a plurality of region designation values so as to calculate a plurality of region identification thresholds;

a classifying device (240) for classifying the plurality of local standard deviations on the basis of the plurality of region identification thresholds;

a selecting device (250) for selecting an image processing parameter for each of categories; and

a processing device (260) for performing image processing on image data, which is contained in the digital image information and of which local standard deviation is designated with the category, using the image processing parameter.

3. The image processing system (40) according to Claim 2, wherein the image processing parameter refers to weight coefficients that define a smoothing filter which smoothes pixel values.

4. The image processing system (40) according to Claim 2 or 3, wherein the working-out device (220) works out an overall standard deviation that is a local standard deviation of a predefined number of pixels equivalent to a total number of pixels contained in the digital image information.

5. The image processing system (40) according to Claim 4, wherein the calculating device (230) calculates a boundary identification threshold on the basis of the overall standard deviation and the smallest value.

6. The image processing system (40) according to Claim 5, wherein the image processing parameter refers to weight coefficients that define a sharpening filter which sharpens pixel values.

7. The image processing system (40) according to Claim 6, wherein the selecting device (250) selects based on the boundary identification threshold whether the image processing parameter for each category defines a smoothing filter or a sharpening filter or defines no change in pixel values.

8. The image processing system (40) according to Claim 7, wherein when the selecting device (250) selects an image processing parameter defining a sharpening filter as the image processing parameter for each category, the selecting device (250) designates the weight coefficients for a category of a local standard deviation exceeding the largest value among the plurality of region identification thresholds.

9. The image processing system (40) according to any of Claims 3 to 8, wherein the weight coefficients are normalized by the sum total of all weight coefficients specified in a kernel of a smoothing filter or a sharpening filter.

10. An X-ray CT system comprising:

a scanner gantry (2) that irradiates an X-ray beam to a subject so as to acquire projection data from the subject; and

a scanner console (6) that reconstructs an image using the projection data so as to produce digital image information representing the subject, wherein:

the scanner console includes an image processing system (40) comprising: a producing device (210) for producing a graph of a cumulative distribution function that provides a cumulative number of pixels, which is the number of pixels that are contained in digital image information and that do not exceed a certain pixel value, for each pixel value; a working-out device (220) for working out a local standard deviation, which is a standard deviation of values of local pixels belonging to an investigation domain including a predefined number of pixels determined with the cumulative numbers of pixels, using the graph of the cumulative distribution function, and repeating the working-out while shifting the investigation domain so that the investigation domain will cover all the cumulative numbers of pixels; a calculating device (230) for detecting the smallest value among the plurality of local standard deviations resulting from the working-out, and multiplying the smallest value by a plurality of region designation values so as to calculate a plurality of region identification thresholds; a classifying device (240) for classifying the plurality of local standard deviations on the basis of the plurality of region identification thresholds; a selecting device (250) for selecting an image processing parameter for each category; and a processing device (260) for performing image processing on image data, which is contained in the digital image information and of which local standard deviation is designated with the category, using the image processing parameter.

Fig. 1

Fig. 2

From operator console 6

200

205

210
(Cumulative number of pixels)

220
(Local (Overall) standard deviation)

230
(Region (Boundary) identification thresholds)

240

250

(Region data)          (Parameter)

260
(Processed image data)

270

40

Fig. 3

(A)

(B)

201

Bronchial and vascular region

Aerial region

Fig. 4

(A)

Number of
pixels

Aerial region

Bronchial and vascular region

$P_1$

$P_2$

Distribution function f(x)

C1    C2    Number of pixels

(B)

Cumulative
number of
pixels

$P_2$

Cumulative distribution
function F(X)

Bronchial and vascular region

$P_1$

C1    C2    Number of pixels

Aerial region

## Fig. 5

## Fig. 6

# Fig. 7

(A)

Weight coefficient

(B)

Weight coefficient

(C)

Weight coefficient

Fig. 8

```
              ┌─────────────┐
              │    START    │
              └──────┬──────┘
                     ↓
              ┌─────────────┐  S901
              │Acquire image│
              │ information │
              └──────┬──────┘
                     ↓
              ┌─────────────┐  S902
              │Delineate region│
              └──────┬──────┘
                     ↓
         ┌──────────────────────┐  S903
         │Produce graph of cumulative│
         │ distribution function │
         └──────────┬───────────┘
                    ↓
              ┌─────────────┐  S904
              │Calculate local│
              │standard deviations│
              └──────┬──────┘
                     ↓
              ┌─────────────┐  S905
              │Calculate region│
              │identification thresholds│
              └──────┬──────┘
                     ↓
              ┌─────────────┐  S906
              │Classify pixels│
              └──────┬──────┘
                     ↓
              ┌─────────────┐  S907
              │Determine weight│
              │ coefficients │
              └──────┬──────┘
                     ↓
              ┌─────────────┐  S908
              │Perform image│
              │ processing  │
              └──────┬──────┘
                     ↓
              ┌─────────────┐  S909
              │Display image│
              └──────┬──────┘
                     ↓
              ┌─────────────┐
              │     END     │
              └─────────────┘
```

Fig. 9

Overall standard deviation

Local standard deviation

0

Investigation domain

Total number of pixels

Cumulative number of pixels

Fig. 1 0

```
        ( START )
            |
    +----------------+
    | Acquire image  |  S401
    | information    |
    +----------------+
            |
    +----------------+
    | Delineate region|  S402
    +----------------+
            |
    +----------------+
    | Calculate cumulative |  S403
    | number of pixels     |
    +----------------+
            |
    +--------------------------+
    | Calculate local standard deviation |  S404
    | and overall standard deviation     |
    +--------------------------+
            |
    +----------------+
    | Calculate region      |  S405
    | identification thresholds |
    +----------------+
            |
    +----------------+
    | Calculate boundary    |  S406
    | identification threshold |
    +----------------+
            |
    +----------------+
    | Classify pixels |  S407
    +----------------+
            |
    +--------------------------+
    | Determine weight coefficients |  S408
    | that define smoothing filter  |
    +--------------------------+
            |
          ( P )
```

Fig. 1 1

```
                    ( P )
                      │
         S409         ▼
           ╲      ╱ ─────── ╲      NO
            ╲   ╱    Is       ╲──────────┐
             ╳  boundary region ╳        │
            ╱   ╲   found?    ╱          │
           ╱      ╲ ───────  ╱           │
                      │ YES              │
         S410         ▼                  │
           ╲      ╱ ───────── ╲   NO     │
            ╲   ╱ Does local standard ╲──┤
             ╳  deviation exceed largest ╳│
            ╱    region identification  ╱ │
           ╱   ╲    threshold?    ╱       │
                  ╲ ───────── ╱          │
                      │ YES              │
         S411         ▼                  │
           ╲  ┌─────────────────────┐    │
            ╲ │  Select weight coefficients │  │
              │ that define sharpening filter│  │
              └─────────────────────┘    │
                      │                  │
                      ▼◄─────────────────┘
         S412 ┌─────────────────┐
           ╲  │  Perform image   │
              │   processing     │
              └─────────────────┘
                      │
         S413         ▼
           ╲  ┌─────────────────┐
              │  Display image   │
              └─────────────────┘
                      │
                      ▼
                ( END )
```

European Patent Office

## EUROPEAN SEARCH REPORT

Application Number

EP 05 25 2905

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 0 653 726 A (EASTMAN KODAK COMPANY) 17 May 1995 (1995-05-17)<br>* abstract; figures 4,7-12 *<br>* column 2, lines 20-44 *<br>* column 5, line 5 - column 6, line 6 *<br>----- | 1-10 | G06T5/40 |
| A | EP 0 794 513 A (SAMSUNG ELECTRONICS CO., LTD) 10 September 1997 (1997-09-10)<br>* page 2, line 3 - page 3, line 49; figures 1,2 *<br>----- | 1-10 | |
| A | EP 1 341 124 A (EASTMAN KODAK COMPANY) 3 September 2003 (2003-09-03)<br>* abstract; figures 1,2,4 *<br>* paragraphs [0001] - [0013] *<br>----- | 1-10 | |
| P,A | GB 2 395 263 A (* QINETIQ LIMITED) 19 May 2004 (2004-05-19)<br>* abstract; figures 2,3,5-8 *<br>* page 16, line 7 - page 18, line 16 *<br>----- | 1-10 | |

TECHNICAL FIELDS SEARCHED (Int.Cl.7)

G06T

The present search report has been drawn up for all claims

| Place of search | Date of completion of the search | Examiner |
|---|---|---|
| Munich | 27 September 2005 | Lahorte, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 25 2905

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2005

| Patent document cited in search report | | Publication date | Patent family member(s) | | Publication date |
|---|---|---|---|---|---|
| EP 0653726 | A | 17-05-1995 | DE | 69427567 D1 | 02-08-2001 |
| | | | DE | 69427567 T2 | 16-05-2002 |
| | | | JP | 3619270 B2 | 09-02-2005 |
| | | | JP | 7192119 A | 28-07-1995 |
| | | | US | 5426684 A | 20-06-1995 |
| EP 0794513 | A | 10-09-1997 | CN | 1164803 A | 12-11-1997 |
| | | | DE | 69717517 D1 | 16-01-2003 |
| | | | DE | 69717517 T2 | 24-04-2003 |
| | | | ES | 2188862 T3 | 01-07-2003 |
| | | | JP | 2858742 B2 | 17-02-1999 |
| | | | JP | 10003538 A | 06-01-1998 |
| | | | KR | 207660 B1 | 15-07-1999 |
| | | | US | 5857033 A | 05-01-1999 |
| EP 1341124 | A | 03-09-2003 | DE | 60300462 D1 | 12-05-2005 |
| | | | JP | 2003296729 A | 17-10-2003 |
| | | | US | 2003161545 A1 | 28-08-2003 |
| GB 2395263 | A | 19-05-2004 | AU | 2003280023 A1 | 03-06-2004 |
| | | | EP | 1563457 A2 | 17-08-2005 |
| | | | WO | 2004044845 A2 | 27-05-2004 |

EPO FORM P0459

For more details about this annex : see Official Journal of the European Patent Office, No. 12/82